# EUROPÄISCHE PATENTSCHRIFT

(11) **EP 0 350 437 B1**
(45) Veröffentlichungstag und Bekanntmachung des Hinweises auf die Patenterteilung: **06.09.1995**
(21) Anmeldenummer: 89730158.6
(22) Anmeldetag: 06.07.1989
(51) Int. Cl.: C07D 207/26, A61K 31/40, C07D 405/10, C07D 409/10

(54) **3,4-Disubstituierte Phenyl-Heterocyclen und deren Verwendung**
3,4-Disubstituted phenyl-heterocycles and their use
Phénylhétérocycles disubstitués en 3,4 et leur utilisation

(30) Priorität: 07.07.1988 DE 3823299
(43) Veröffentlichungstag der Anmeldung: 10.01.1990
(73) Patentinhaber: SCHERING AKTIENGESELLSCHAFT, 13342 Berlin (DE)
(72) Erfinder: Huth, Andreas, Dr., D-1000 Berlin 38 (DE); Schmiechen, Ralph, Dr., D-1000 Berlin 42 (DE); Wachtel, Helmut, Dr., D-1000 Berlin 19 (DE); Schneider, Herbert Hans, Dr., D-1000 Berlin 15 (DE)

(56) Entgegenhaltungen:
- AT-B- 310 150
- GB-A- 1 552 686
- US-A- 4 186 129
- US-A- 4 193 926
- CHEMICAL ABSTRACTS, Band 105, Nr. 14, 6. Oktober 1986, Columbus, Ohio, USA , KLOSE WALTER et al. "Oxa- zolidinone and pyrrolidone derivatives as inflammation inhibitors" Seite 372, Spalte 2, Zusammenfassung-Nr. 120 765m & Ger. Offen DE3,438,839

## Beschreibung

Die Erfindung betrifft neue Pyrrolidinon-, Oxazolidinon- und Imidazolidinon-Derivate, deren Herstellung und diese enthaltende Arzneimittel.

Im US-Patent 4.193.926 werden 4-Polyalkoxyphenyl-2-pyrrolidinone und im US-Patent 4.186.129 und der PCT/DE 85/00472 werden 5-Polyalkoxyphenyl-oxazolidinnne und im EP-A-0247725 werden 5-(3-Polycycloalkoxy-4-alkoxyphenyl-imidazolidinone beschrieben. Die Verbindungen zeigen neuropsychotrope u.a. neuroleptische und antidepressive Eigenschaften wobei sich die in der EP-A-0247725 beschriebenen Imidazolidinone durch einen günstigen therapeutischen Index und durch verminderte Emesis auszeichnen.

Die erfindungsgemäßen Verbindungen, die überraschenderweise über einen längeren Zeitraum besser bio-verfügbar sind und sich durch geringe gastrointestinale Nebenwirkungen auszeichnen, haben die allgemeine Formel I 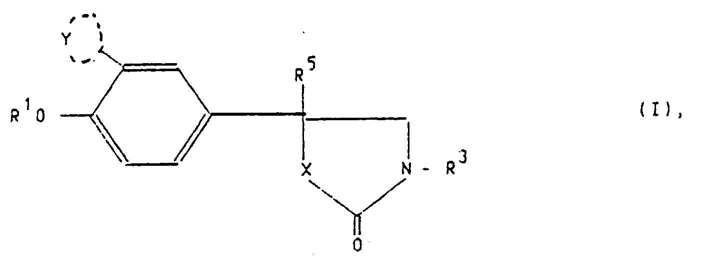
worin
R¹ C₁₋₄-Alkyl,
R³ Wasserstoff, C₁₋₄-Alkyl, Acyl, Phenyl
R⁵ Wasserstoff, C₁₋₄-Alkyl,
X Sauerstoff, CH₂ oder NR⁴ ist, mit R⁴ = Wasserstoff, C₁₋₄-Alkyl
und
Y ein monocyclisches oder bicyclisches, aromatisches oder nicht aromatisches Ringsystem, mit 3 - I2 Ringatomen, das I - 2 Schwefel-, Sauerstoff- und/oder Stickstoffatome enthalten kann und substituiert sein kann mit Halogen, C_{I-4}-Alkyl, NH₂ oder OR², worin R² Wasserstoff, C_{I-4}-Alkyl, C₃₋₇-Cycloalkyl oder einen cyclischen Etherrest bedeutet und die Stereoisomeren und deren Gemische.

Da die Verbindungen der allgemeinen Formel I mindestens ein asymmetrisches Zentrum besitzen, umfassen diese alle möglichen Stereo-Isomeren und Gemische davon (Diastereomere als Racemate und Enantiomere).

Das aromatische oder nicht aromatische Ringsystem enthält 3 - 12 Ringatome, vorzugsweise 5 - 9 Ringatome und kann monocyclisch oder bicyclisch sein.

Unter einem aromatischen Ring ist sowohl der Aryl- wie auch der Hetaryl-Rest zu verstehen, wobei beispielsweise die folgenden Mono- oder Bicyclen genannt seien: Aryl wie Phenyl, Biphenylyl, Indenyl, Naphthyl und Hetaryl mit 1 - 2 Heteroatomen wie Schwefel, Sauerstoff und/oder Stickstoff wie Thienyl, Furyl, Pyranyl, Pyrrolyl, Imidazolyl, Pyrazolyl, Pyridyl, Pyrimidinyl, Pyridazinyl, Oxazolyl, Iso-oxazolyl, Thiazolyl, Isothiazolyl, Benzo[1]thienyl, Benzofuryl. Als bevorzugt sind 5 - 6 gliedrige aromatische Ringsysteme anzusehen, die gegebenenfalls ein Heteroatom enthalten.

Unter einem nicht aromatischen Ringsystem sind gesättigte und ungesättigte alicyclische und aryl-alicyclische Reste zu verstehen, die mono- oder bicyclisch sein können und wobei die Ringe kondensiert, spiro-verknüpft oder isoliert sein können. Beispielsweise seien genannt: Cycloalkyl, Cycloalkenyl, Phenylcycloalkyl, Bicycloalkyl und Bicycloalkenyl wie Cyclopropyl, Cyclobutyl, Cyclopentyl, Cyclohexyl, Cycloheptyl, Cyclooctyl, Cyclohexenyl, Cyclopentenyl, Cycloheptenyl, Bicycloheptyl, Bicycloheptenyl, Indan-2-yl, Inden-2-yl. Als Heteroatome für das nicht aromatische Ringsystem kommen 1 - 2 Stickstoff-, Sauerstoff- und/oder Schwefelatome in Betracht, wobei die oben genannten Hetaryle teilweise oder vollständig hydriert sein können. Beispielsweise seien genannt: Pyrrolidinyl, Thiazolidinyl, Morpholinyl, Tetrahydrofuranyl, Tetrahydropyranyl. Bevorzugte nicht aromatische Ringsysteme sind C₃₋₇-Cycloalkyl, C₃₋₇-Cycloalkenyl, Norbornan, 1-Hydroxy-C₃₋₇-Cycloalkyl, Phenylcycloalkyl wie Indan und Inden. Ist das Ringsystem substituiert, so ist insbesondere der Aromat mono-oder di-substituiert durch Halogen, C₁₋₄-Alkyl, NH₂ und OR², wobei unter R² Wasserstoff, C₁₋₄-Alkyl, C₃₋₇-Cycloalkyl und cyclische Ether wie Tetrahydrofuranyl und Tetrahydropyranyl zu verstehen sind.

Halogen kann für Fluor, Chlor, Brom und Jod stehen.

Unter C₁₋₄-Alkyl ist jeweils Methyl, Ethyl, Propyl, Isopropyl, Butyl, Isobutyl, sek. Butyl und tert. Butyl gemeint.
Die Arylgruppe R³ ist bevorzugt Phenyl.
Als Acylreste R³ können alle im US-Patent 4186129 genannten organischen Säuren verwendet werden; insbesondere kommen jedoch niedere Alkanoylgruppen mit bis zu 4 Kohlenstoffatomen in Betracht wie Formyl, Acetyl, Propionyl, Butyryl.

In Arzneimitteln bevorzugte Verbindungen sind solche der allgemeinen Formel I, worin R¹ Methyl, R³ Wasserstoff und X CH₂ oder 0 darstellt.

Die Herstellung der Verbindungen der allgemeinen Formel I erfolgt nach an sich bekannten Verfahren, indem man beispielsweise die Verbindungen der allgemeinen Formel II 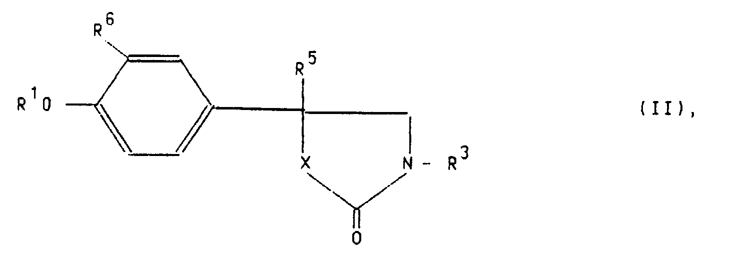
worin R¹, R³, R⁵ und X die obige Bedeutung haben und
R⁶ eine Flucht-Gruppe ist
a)
   in Gegenwart eines Palladiumkatalysators mit 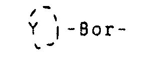 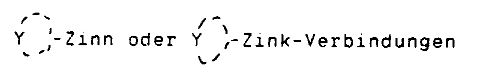 oder mit Cycloalkenen umsetzt, wobei Y die oben genannte Bedeutung hat oder
b)
   in ein Lithium-Organyl überführt und mit dem entsprechenden Keton umsetzt zu 1-Hydroxy-Cycloalkyl und gegebenenfalls durch Wasserabspaltung das Cycloalken darstellt
und gewüschtenfalls anschließend Doppelbindungen hydriert und/oder NH-Gruppen alkyliert, aryliert oder acyliert und/oder Etherschutzgruppen abspaltet und gegebenenfalls anschließend verethert und/oder die Isomeren auftrennt.

Als Flucht-Gruppen sind Halogene, insbesondere Brom und Jod, sowie Sulfonate wie Trifluormethansulfonat geeignet.

Als Palladium-Katalysatoren werden vorzugsweise organische Palladium-II-Salze wie Palladium-acetat oder -chlorid gegebenenfalls in Gegenwart eines Liganden wie Triphenylphosphin oder Tris-ortho-Tolylphosphin oder Palladium(O)-Komplexe wie z.B. Palladium-tetrakistriphenylphosphin in katalytischen oder größeren Mengen verwendet.

Die Umsetzung kann durch Basen beschleunigt werden. Als Basen kommen sekundäre und tertiäre Amine wie Triethylamin und anorganische Basen wie Alkali- und Erdalkali-Hydroxide und -carbonate wie Natrium-, Kalium und Lithiumhydroxid und Thalliumhydroxid in Betracht Der Zusatz von Lithiumchlorid hat bei der Umsetzung von Triflaten einen positiven Einfluß.

Die anorganischen Basen können in Alkoholen oder Wasser gelöst werden, wobei der Phasentransfer durch die üblichen Phasentransferkatalysatoren beschleunigt werden kann.

Als Bor-, Zinn- und Zink-Derivate können z.B. organische Boronsäuren, organische Zinkchloride und Tri-C₁₋₄-Alkyl-Zinn-Verbindungen eingesetzt werden.

Die Einführung des Ringsystems erfolgt in geeigneten Lösungsmitteln oder Lösungsmittelgemischen bei Temperaturen von Raumtemperatur bis zur Siedetemperatur der Lösung und ist nach ca. 1 - 5 Stunden beendet.

Als Lösungsmittel geeignet sind unpolare Lösungsmittel wie Toluol, Xylol und aprotisch polare Lösungsmittel wie Dimethylformamid, Dimethylsulfoxid, die gegebenenfalls auch in Gemischen mit protischen Lösungsmitteln wie Alkoholen (Methanol, Ethanol u.a. ) eingesetzt werden können.

Zur Überführung in das Lithium-Organyl sind beispielsweise Lithiumalkyle, wie insbesondere n-Butyllithium,tert. Butyllithium und Lithiumphenyl geeignet. Die Umsetzung wird in aprotischen Lösungsmitteln wie Ethern oder Kohlenwasserstoffen, beispielsweise THF, Dioxan, Diethylether, Toluol, Hexan u.a., bei Temperaturen von 20 °C bis -110 °C, vorzugsweise 20 °C bis - 78 °C vorgenommmen. Zur Vermeidung von Nebenreaktionen kann das Proton am Stickstoff mit den üblichen Schutzgruppen ersetzt werden, wie beispielsweise durch einen Acyl- oder Silylrest wie die Trialkylsilyl- insbesondere die tert. Butyldimethylsilylgruppe, die nach Beendigung der Reaktion nach den üblichen Methoden wie beispielsweise durch Behandeln mit Sauren, wie verdünnter Mineralsäure, Trifluoressigsäure oder auch anorganischen Basen wie Alkalihydroxid oder Fluoriden wie Tetrabutylammoniumfluorid bei Raumtemperatur abgespalten werden. Die Reaktion ist nach ca. 5 Minuten bis zu 2 Stunden beendet; gegebenenfalls kann 1 - 2 Stunden bei Raumtemperatur nachgerührt werden. Zweckmäßigerweise arbeitet man unter Schutzgasatmosphäre wie Argon oder Stickstoff.

Die Umsetzung mit dem Keton wird in den oben genannten aprotischen Lösungsmitteln wie Ethern und Kohlenwasserstoffen bei tiefen Temperaturen von 0 °C bis - 78 °C durchgeführt, wobei anschließend bei 0 °C bis Raumtemperatur nachgerührt wird. Die Wasserabspaltung erfolgt nach den üblichen Verfahren wie beispielsweise mit Säuren wie Mineralsaure, Thionylchlorid/Pyridin oder Zugabe von wasserfreiem Kupfersulfat.

Die Hydrierung von Doppelbindungen wird vorzugsweise katalytisch zum Beispiel mit Edelmetallkatalysatoren wie Platin oder Palladium gegebenenfalls auf geeigneten Trägern wie Kohle in protischen Lösungsmitteln wie Alkoholen beispielsweise Methanol, Ethanol u.a. bei Raumtemperatur bis zur Siedetemperatur des Lösungsmittels unter Normaldruck oder H₂-Druck vorgenommen.

Alkylierung, Arylierung oder Acylierung einer Iminogruppe erfolgen nach bekannten Methoden. Beispielsweise wird die Iminoverbindung in einem polaren Lösungsmittel gelöst und in Gegenwart einer Base mit einem Alkyl-, Aryl- oder Acylhalogenid auf etwa 40 bis 50 °C erhitzt. Als polare Lösungsmittel kommen beispielsweise Dimethylformamid, Dimethylacetamid, cyclische Ether wie Tetrahydrofuran, Dioxan, Alkohole wie Ethanol, Methanol, Propanol und als Basen zum Beispiel Natriumhydrid, Alkalialkoholate wie Natriumethylat in Betracht. Die Umsetzung mit Halogenaryl, beispielsweise Jodbenzol, kann auch ohne Lösungsmittel, vorzugsweise in Gegenwart von Kupferpulver, durchgeführt werden.

Zweckmäßigerweise werden freie Hydroxygruppen vor der Einführung des Ringsystems mit den üblichen Etherschutzgruppen wie beispielsweise dem Tetrahydrofuranyl-, Tetrahydropyranyl-Rest geschützt, der nach Beendigung der Umsetzung nach gebrächlichen Methoden beispielsweise mit Säuren abgespalten werden kann. Für die Abspaltung geeignete Säuren sind anorganische oder organische Säuren wie beispielsweise Pyridiniumtosylat, HCl, p-Toluolsulfonsäure. Die Abspaltung wird üblicherweise in protischen Lösungsmitteln wie Alkoholen (Methanol, Ethanol oder Aceton) bei Raumtemperatur bis zur Siedetemperatur des Lösungsmittels vorgenommen.

Die anschließende Veretherung kann auch nach bekannten Verfahren erfolgen wie beispielsweise durch Umsetzung mit einem entsprechenden Tosylat oder Halogenid wie Chlorid, Bromid oder Jodid in Gegenwart einer Base wie gepulverter KOH oder Tetrabutylammoniumhydrogensulfat. Die Veretherung kann in protischen Lösungsmitteln wie Alkoholen beispielsweise Methanol, Ethanol, Propanol oder aprotisch dipolaren Lösungsmitteln wie Dimethylformamid, Dimethylsulfoxid, Tetrahydrofuran bei Raumtemperatur bis zur Siedetemperatur des Lösungsmittels durchgeführt werden.

Die Isomerentrennung kann mit bekannten Methoden wie beispielsweise Kristallisation, Salzbildung und Chromatographie vorgenommen werden.

Die neuen Verbindungen der Formel I sind pharmakologisch wirksame Substanzen. die sich auf Grund ihrer biologischen Wirksamkeit insbesondere als Psychopharmaka für die Humanmedizin eignen. Ihr Wirkmechanismus beruht auf einer Verstarkung der Neurotransmission jenseits von Transmitterrezeptoren auf Grund einer cyclo-Adenosinmonophosphat- und Phosphodiesterase-Hemmung, wodurch insbesondere affektive und cognitive Störungen bei neurodegenerativen Erkrankungen beeinflußt werden. Anticholinerge Nebenwirkungen wie kardiovaskuläre Nebenwirkungen oder Augeninnendruckerhöhung bei Glaukompatienten treten nicht auf. Die erfindungsgemäßen Verbindungen eignen sich daher zur Behandlung präseniler und seniler Demenz, Morbus Parkinson, Altersdepression und Demenz infolge Alkoholmißbrauchs.

Aufgrund ihres Wirkprofils zeigen Verbindungen der allgemeinen Formel I auch periphere Wirkungen und besitzen antiinflammatorische, bronchospasmolytische, antiproliferative, thrombocytenaggregationshemmende und tokolytische Eigenschaften.

Sie können zu Präparationen, zum Beispiel für die orale und parenterale Anwendung, formuliert werden.

Als Formulierungshilfsstoffe sind physiologisch verträgliche organische und anorganische Trägersubstanzen geeignet, die gegenüber den erfindungsgemäßen Verbindungen inert sind.

Als Tragersubstanzen seien beispielsweise genannt Wasser, Salzlösungen, Alkohole, Polyäthylenglykole, polyhydroxyethoxyliertes Rizinusöl, Gelatine, Lactose, Amylose, Magnesiumstearat, Talkum, Kieselsäure, Fettsäure-mono- und -diglyceride, Pentaerythritolfettsäureester, Hydroxymethylcellulose und Polyvinylpyrrolidon.

Die pharmazeutischen Präparationen können sterilisiert werden und/oder mit Hilfsstoffen, wie Schmiermittel, Konservierungsstoffen, Stabilisatoren, Netzmittel, Emulgatoren, Puffermittel und Farbstoffen, versetzt werden.

Für die parenterale Anwendung sind insbesondere Injektionslösungen oder Suspensionen, insbesondere wäßrige Losungen der aktiven Verbindungen in polyhydroxyethoxyliertem Rizinusöl, geeignet. Als Trägersysteme können auch grenzflachenaktive Hilfsstoffe wie Salze der Gallensäuren oder tierische oder pflanzliche Phospholipide, aber auch Mischungen davon sowie Liposome oder deren Bestandteile verwendet werden.

Für die orale Anwendung sind insbesondere Tabletten, Dragees oder Kapseln mit Talkum und/oder einem Kohlenwasserstofftrager oder -binder, wie zum Beispiel Lactose, Mais- oder Kartoffelstarke, geeignet. Die Anwendung kann auch in flussiger Form erfolgen, wie zum Beispiel als Saft, dem gegebenenfalls ein Süßstoff beigefügt wird.

Die erfindungsgemäßen Verbindungen werden in einer Dosiseinheit von 0,1 bis 50 mg aktiver Substanz in einem physiologisch verträglichen Träger eingebracht.

Die erfindungsgemäßen Verbindungen werden in einer Dosis von 0,1 bis 50 mg/Tag, vorzugsweise 1 - 10 mg/Tag, angewendet.

Die Ausgangsverbindungen sind bekannt oder können nach an sich bekannten Verfahren dargestellt werden. So können 3-unsubstituierte 4-alkoxy-Phenylderivate in üblicher Weise halogeniert werden, beispielsweise mit elementarem Halogen oder Halogensäuren oder das 3-OH-substituierte Phenyl-Derivat wird in üblicher Weise trifluormethansulfonyliert. Die Ausgangsverbindungen sind beispielsweise in den vorne genannten Patenten beschrieben.

Die Zink-, Zinn- oder Bor-Organyle können z.T. gekauft oder beispielsweise nach der in Houben Weyl Bd. 13/6 (Zinn), Bd. 13/3a und 3b (Bor) sowie J. Org. Chemie 42, 1821 (1977) (Zink) beschriebenen Methode erhalten werden. Die nachfolgend beschriebenen Ausgangsverbindungen und Beispiele sollen das erfindungsgemäße Verfahren erläutern.

### Beispiel A

### 4-(4-Methoxy-3-trifluormethansulfonyloxyphenyl)-pyrrolidin-2-on

10,35 g (50 mMol) 4-(4-Methoxy-3-hydroxyphenyl)-pyrrolidin-2-on werden in 500 ml Methylenchlorid unter Feuchtigkeitsausschluß und Schutzgas vorgelegt, mit 20 g Dimethylaminopyridin versetzt und auf 0 °C gekühlt. Anschließend wird eine Lösung von 8,7 ml Trifluormethansulfonsäureanhydrid in 100 ml Methylenchlorid langsam zugetropft und 1/2 Stunde bei 0 °C nachgerührt. Der Ansatz wird anschließend mit 300 ml Wasser und 200 ml 1-N-Salzsäure versetzt und extrahiert. Die Methylenchloridphase wird nacheinander mit 300 ml 1-N-Salzsäure und 300 ml Wasser gewaschen, getrocknet, filtriert und eingeengt. Die Umkristallisation des Rückstandes aus Essigester/Hexan ergibt 14,35 g (85 % der Theorie). Man erhält 4-(4-Methoxy-3-trifluormethansulfonyloxyphenyl)-pyrrolidin-2-on vom Schmelzpunkt 99 - 101 °C.

### Beispiel B

### 4-(3-Jod-4-methoxyphenyl)-pyrrolidin-2-on

Eine Lösung von 19,1 g 4-(4-Methoxyphenyl)-pyrrolidin-2-on (100 mMol) in 200 ml Eisessig, 4,8 ml Wasser und 1,2 ml konzentrierter Schwefelsäure wird mit 3,44 g (19,55 mMol) Jodsäure und 8,64 g (70 mMol) Jod versetzt und 10 Stunden bei 80 °C Badtemperatur gerührt. Anschließend wird am Rotationsverdampfer bis zur beginnenden Kristallisation eingeengt, abgekühlt, der krist. Niederschlag abgesaugt und aus Essigester umkristallisiert.
Man erhalt 25,7 g (81 % der Theorie) 4-(3-Jod-4-methoxyphenyl)-pyrrolidin-2-on vom Schmelzpunkt 158 °C.

### Beispiel C

### 4-(3-Brom-4-Methoxyphenyl)-pyrrolidin-2-on

190 mg (1 mMol) 4-Methoxyphenylpyrrolidin-2-on werden in 1 ml Eisessig gelöst und bei Raumtemperatur mit 160 mg Brom in 0,5 ml Eisessig tropfenweise versetzt. Nach 1 Stunde Rühren wird mit Wasser verdünnt. Nach Absaugen und Trocknen erhält man 220 mg (81 % der Theorie) 4-(3-Brom-4-methoxyphenyl)pyrrolidin-2-on vom Schmelzpunkt 151 - 154 °C.

### Beispiel D

### 4-(3-Brom-4-methoxyphenyl)-1-t-butyldimethylsilylpyrrolidin-2-on

540 mg (2mMol) 4-(3-Brom-4-methoxyphenyl)-pyrrolidin-2-on werden in 10 ml Dimethylformamid unter Stickstoff mit 60 mg (2mMol) Natriumhydrid versetzt. Nach 20 Minuten Rühren werden nach Abkühlen auf 0 °C 300 mg (2 mMol) tert.Butyl-dimethylsilylchlorid in 5 ml Dimethylformamid zugetropft und 30 Minuten bei 0 °C sowie 30 Minuten bei Raumtemperatur gerührt. Nach Einengen wird in Essigester/Wasser verteilt. Die Essigesterphase wird getrocknet, filtriert und eingeengt und der Rückstand über Kieselgel mit Methylenchlorid : Ethanol = 10 : 1 als Elutionsmittel chromatographiert. Man erhält 420 mg 4-(3-Brom-4-methoxyphenyl)-1-t-butyldimethylsilylpyrrolidin-2-on vom Schmelzpunkt 150 - 152 °C.

### Beispiel 1

### 4-(2,3'-Dimethoxy-5-biphenylyl)-pyrrolidin-2-on

339 mg 4-(4-Methoxy-3-trifluormethansulfonyloxyphenyl)-pyrrolidin-2-on werden in 9 ml Toluol und 4 ml Ethanol gelöst und 5' unter Argon bei Raumtemperatur mit 38 mg Tetrakis(triphenylphosphin)palladium(0) gerührt. Nach Zugabe von 172 mg m-Methoxyphenyl-boronsäure und 1,3 ml 2m-Natriumcarbonat-Lösung wird 4 Stunden auf 90 °C erhitzt. Nach Zusatz von Essigester und Wasser wird die organische Phase abgetrennt, mit gesättigter Natriumchloridlösung neutral gewaschen, über Natriumsulfat getrocknet und eingeengt. Durch Umkristallisieren aus Alkohol/Essigester/Petrolether (40 - 60 °C) erhält man 187 mg Substanz vom Schmelzpunkt 137 - 139 °C.

Die folgenden Verbindungen werden in analoger Weise hergestellt:
4-[2-Methoxy-3'-(tetrahydropyran-2-yloxy)-5-biphenylyl]-pyrrolidin-2-on als Öl.
4-(3',5'-Dichlor-6-methoxy-3-biphenylyl)-pyrrolidin-2-on
Schmelzpunkt 162 - 164 °C.
4-(2-Methoxy-5-biphenyl-yl)-pyrrolidin-2-on
Schmelzpunkt 153 - 154 °C.
4-(2-Methoxy-4'-methyl-5-biphenylyl)-pyrrolidin-2-on
Schmelzpunkt 146 - 147 °C.
4-(2,4'-Dimethoxy-5-biphenylyl)-pyrrolidin-2-on
Schmelzpunkt 169 °C.
4-[4-Methoxy-3-(2-furyl)-phenyl]-pyrrolidin-2-on
Schmelzpunkt 152 - 153 °C.
4-[4-Methoxy-3-(2-thienyl)-phenyl]-pyrrolidin-2-on
Schmelzpunkt 125 °C.
4-[4-Methoxy-3-(3-thienyl)-phenyl]-pyrrolidin-2-on
Schmelzpunkt 135 - 136 °C.
4-(2',4'-Dichlor-6-methoxy-3-biphenylyl)-pyrrolidin-2-on
Schmelzpunkt 145 - 146 °C.
4-(2,2'-Dimethoxy-5-biphenylyl)-pyrrolidin-2-on
Schmelzpunkt 116 - 117 °C.
4-(4'-Chlor-6-methoxy-3-biphenylyl)-pyrrolidin-2-on
Schmelzpunkt 162 - 163 °C.
4-(3'-Chlor-2-methoxy-5-biphenylyl)-pyrrolidin-2-on
Schmelzpunkt 143 - 144 °C.
4-(2-Methoxy-3'-methyl-5-biphenylyl)-pyrrolidin-2-on
Schmelzpunkt 159 - 160 °C.

### Beispiel 2

### 4-(3-Hydroxy-2-methoxy-5-biphenylyl)-pyrrolidin-2-on

894 mg 4-[2-Methoxy-3'-(tetrahydropyran-2-yloxy)-5-biphenylyl]-2-pyrrolidin-2-on werden in 10 ml Ethanol und 2 ml Wasser gelöst und mit 754 mg Pyridinium-p-toluolsulfonat 1 Stunde am Rückfluß erhitzt. Nach Abdestillieren des Lösungsmittels wird das Reaktionsprodukt in Essigester aufgenommen und die Lösung nacheinander mit gesättigter Natriumhydrogencarbonat-Lösung sowie mit gesättigter Natriumchlorid-Lösung gewaschen, über Natriumsulfat getrocknet, filtriert und eingeengt. Nach Säulenchromatographie über 150 g Kieselgel (40-63µ) mit Methylenchlorid/Ethanol 10 : 1 als Elutionsmittel isoliert man 464 mg 4-(3'-Hydroxy-2-methoxy-5-biphenylyl)-pyrrolidin-2-on als Öl.

### Beispiel 3

### 4-(3'-Cyclopentyloxy-2-methoxy-5-biphenylyl)-pyrrolidin-2-on

100 mg 4-(3'-Hydroxy-2-methoxy-5-biphenylyl)-2-pyrrolidin-2-on und 90 Mikroliter Cyclopentylbromid werden in 10 ml absolutem Ethanol mit 70 mg Kaliumhydroxid-Pulver 3 Stunden am Rückfluß erhitzt. Nach dem Einengen des Ansatzes wird in Essigester aufgenommen, mit gesättigter Natriumchlorid-Lösung gewaschen, über Natriumsulfat getrocknet, filtriert und eingeengt. Aus dem Rückstand erhält man durch Säulenchromatographie an 75 g Kieselgel (40-63µ) mit Methylenchlorid/Ethanol 10 : 1 als Elutionsmittel 79 mg 4-(3'-Cyclopentyloxy-2-methoxy-5-biphenylyl)-pyrrolidin-2-on als Schaum.

### Beispiel 4

### Gemisch von 4-[3-(Cyclopent-1-en-1-yl)-4-methoxyphenyl]-pyrrolidin-2-on und 4-[3-(Cyclopent-2-en-1-yl)-4-methoxyphenyl]-pyrrolidin-2-on

4,7 g (14,8 mMol) 4-(3-Jod-4-methoxyphenyl)-pyrrolidin-2-on werden in einem Druckgefäß in 100 ml Dimethylformamid gelöst und mit 2,56 ml Triethylamin, 400 mg Tri-ortho-Tolylphosphin, 170 mg Palladiumacetat und 2,5 ml (28 mMol) Cyclopenten (28 mMol) versetzt. Man läßt die Reaktionslösung unter Argon 24 Stunden bei einer Temperatur von 120 - 125 °C in dem geschlossenen Druckgefäß reagieren. Anschließend wird eingedampft in Essigester aufgenommen und mit gesättigter Natriumchlorid-Lösung gewaschen, über Sikkon getrocknet, filtriert und eingedampft. Der Rückstand von 4,4 g eines braunen Oles wird über Kieselgel mit Methylenchlorid : Ethanol = 1 : 1 als Elutionsmittel chromatographiert und ergibt 3 g (79 % der Theorie) des oben genannten Gemisches als Öl.

In analoger Weise werden hergestellt:
Gemisch aus 4-[3-(Cyclohex-1-en-1-yl)-4-methoxyphenyl]-pyrrolidin-2-on und 4-[3-(Cyclohex-2-en-1-yl)-4-methoxyphenyl]-pyrrolidin-2-on,
Schmelzpunkt 138 °C.
Gemisch aus 4-[3-(Cyclohept-1-en-1-yl)-4-methoxyphenyl]-pyrrolidin-2-on und 4-[3-(Cyclohex-2-en-1-yl)-4-methoxyphenyl]-pyrrolidin-2-on als Öl.
4-[3-(Inden-2-yl)-4-methoxyphenyl]-pyrrolidin-2-on
Schmelzpunkt 163-165°C.

### Beispiel 5

### 4-(3-Cyclopentyl-4-methoxyphenyl)-pyrrolidin-2-on

3,6 g (14 mMol) des Isomerengemisches (Beispiel 4) werden in 100 ml Ethanol mit 250 mg Platindioxid unter Normalbedingungen hydriert. Anschließend wird vom Katalysator abfiltriert und das Filtrat eingedampft. Umkristallisation des Rückstandes aus Essigester ergibt 2,3 g (63 % der Theorie) 4-(3-Cyclopentyl-4-methoxyphenyl)-pyrrolidin-2-on vom Schmelzpunkt 139 °C.

In analoger Weise werden hergestellt:
4-(3-Cyclohexyl-4-methoxyphenyl)-pyrrolidin-2-on
Schmelzpunkt 164 °C.
4-(3-Cycloheptyl-4-methoxyphenyl)-pyrrolidin-2-on
Schmelzpunkt 157 °C.
4-[3-(Indanyl)-4-methoxyphenyl]-2-pyrrolidon
Schmelzpunkt 157 °C
4-[3-(exo-Norborn-2-yl)-4-methoxyphenyl]-2-pyrrolidon
Schmelzpunkt 149-151 °C

### Beispiel 6

### 4-[4-Methoxy-3-(1-hydroxycyclopent-1-yl)-phenyl]-pyrrolidin-2-on

2,2 g (5,7 mMol) 4-(3-Brom-4-methoxyphenyl)-1-t-butydimethysilylpyrrolidin-2-on werden in 30 ml absolutem Tetrahydrofuran bei - 70 °C mit 8,2 ml (0,7 mol) einer Butyllithiumlösung in Hexan versetzt. Man läßt auf 0 °C kommen, rührt 10 Minuten bei dieser Temperatur und kühlt wieder auf - 70 °C ab. Dann tropft man eine Lösung von 0,48 g Cyclopentanon in 10 ml Tetrahydrofuran zu, läßt langsam auf 0 °C kommen und rührt bei dieser Temperatur 1 Stunde. Nach Zufügen von 0,1 ml Wasser wird eingedampft und über Kieselgel mit Methylenchlorid:Aceton = 1 : 1 als Elutionsmittel chromatographiert. Nach nochmaliger Chromatographie der entsprechenden Fraktionen über Kieselgel mit Methylenchlorid: Ethanol = 10 : 1 als Elutionsmittel erhält man 110 mg 4-[4-Methoxy-3-(1-hydroxycyclopent-1-yl)-phenyl]-pyrrolidin-2-on als Öl.

## Patentansprüche

1. Verbindungen der allgemeinen Formel I 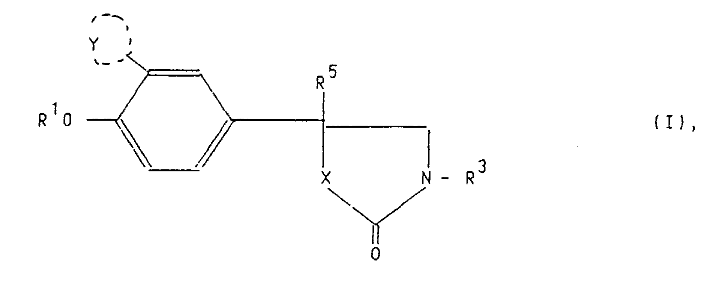 worin
R¹ C₁₋₄-Alkyl,
R³ Wasserstoff, C₁₋₄-Alkyl, Acyl, Phenyl
R⁵ Wasserstoff, C₁₋₄-Alkyl,
X Sauerstoff, CH₂ oder NR⁴ ist, mit R⁴ = Wasserstoff, C₁₋₄-Alkyl
und
Y ein monocyclisches oder bicyclisches aromatisches oder nicht aromatisches Ringsystem, mit 3 - I2 Ringatomen, das I - 2 Schwefel-, Sauerstoff- und/oder Stickstoffatome enthalten kann und substituiert sein kann mit Halogen, C_{I-4}-Alkyl, NH₂ oder OR², worin R² Wasserstoff, C_{I-4}-Alkyl, C₃₋₇-Cycloalkyl oder einen cyclischen Etherrest bedeutet und die Stereoisomeren und deren Gemische.

2. Verbindungen der allgemeinen Formel I gemäß Patentanspruch 1 mit R¹ = CH₃

3. Verbindungen der allgemeinen Formel I gemäß Anspruch 1 mit R³ = H.

4. Verbindungen der allgemeinen Formel I gemäß Anspruch 1 mit X = 0.

5. Verbindungen der allgemeinen Formel I gemäß Anspruch 1 mit X = CH₂.

6. Verbindungen der allgemeinen Formel I gemäß Anspruch 1 und zwar:
4-(2,3'-Dimethoxy-5-biphenylyl)-pyrrolidin-2-on
4-(3',5'-Dichlor-6-methoxy-3-biphenylyl)-pyrrolidin-2-on
4-(2-Methoxy-5-biphenyl-yl)-pyrrolidin-2-on
4-(2-Methoxy-4'-methyl-5-biphenylyl)-pyrrolidin-2-on
4-(2,4'-Dimethoxy-5-biphenylyl)-pyrrolidin-2-on
4-[4-Methoxy-3-[2-furyl)-phenyl]-pyrrolidin-2-on
4-[4-Methoxy-3-(2-thienyl)-phenyl]-pyrrolidin-2-on
4-[4-Methoxy-3-(3-thienyl)-phenyl]-pyrrolidin-2-on
4-(2',4'-Dichlor-6-methoxy-3-biphenylyl)-pyrrolidin-2-on
4-(2,2'-Dimethoxy-5-biphenylyl)-pyrrolidin-2-on
4-(4'-Chlor-6-methoxy-3-biphenylyl)-pyrrolidin-2-on
4-(3'-Chlor-2-methoxy-5-biphenylyl)-pyrrolidin-2-on
4-(2-Methoxy-3'-methoxy-5-biphenylyl)-pyrrolidin-2-on
4-(3'-Cyclopentyloxy-2-methoxy-5-biphenylyl)-pyrrolidin-2-on
4-(3-Cyclopentyl-4-methoxyphenyl)-pyrrolidin-2-on
4-(3-Cyclohexyl-4-methoxyphenyl)-pyrrolidin-2-on
4-(3-Cycloheptyl-4-methoxyphenyl)-pyrrolidin-2-on

7. Verfahren zur Herstellung der Verbindungen der allgemeinen Formel I dadurch, daß man die Verbindungen der allgemeinen Formel II 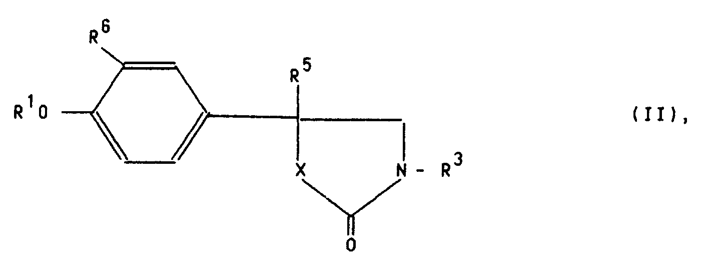 worin R¹, R³, R⁵ und X die obige Bedeutung haben und R⁶ eine Fluchtgruppe darstellt,
a)
in Gegenwart eines Palladiumkatalysators mit einer 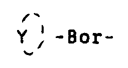 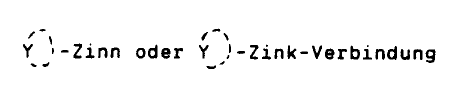 oder mit Cycloalkenen umsetzt, wobei Y die oben genannte Bedeutung hat oder
b)
in ein Lithium-Organyl überführt und dieses mit dem entsprechenden Keton umsetzt zu 1-Hydroxy-Cycloalkyl und gegebenenfalls durch Wasserabspaltung das Cycloalken darstellt
und gewünschtenfalls anschließend Doppelbindungen hydriert und/oder NH-Gruppen alkyliert, aryliert oder acyliert und/oder Etherschutzgruppen abspaltet und gegebenenfalls anschließend verethert und/oder die Isomeren auftrennt.

8. Arzneimittel auf Basis der Verbindungen gemäß den Ansprüchen 1-6.

## Claims

1. Compounds of the general formula I 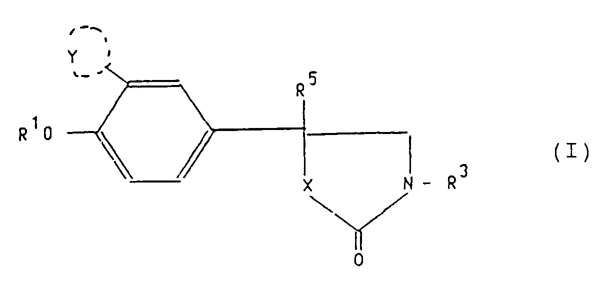 wherein
R¹ is C₁₋₄-alkyl,
R³ is hydrogen, C₁₋₄-alkyl, acyl or phenyl,
R⁵ is hydrogen or C₁₋₄-alkyl,
X is oxygen, CH₂ or NR⁴, wherein R⁴ = hydrogen or C₁₋₄-alkyl,
and
Y is a monocyclic or bicyclic, aromatic or non-aromatic ring system having from 3 to 12 ring atoms, which may contain 1 or 2 sulphur, oxygen and/or nitrogen atoms and may be substituted by halogen, C₁₋₄-alkyl, NH₂ or by OR², wherein R² is hydrogen, C₁₋₄-alkyl, C₃₋₇-cycloalkyl or a cyclic ether radical, and the stereoisomers and mixtures thereof.

2. Compounds of the general formula I according to patent claim 1 wherein R¹ = CH₃.

3. Compounds of the general formula I according to claim 1 wherein R³ = H.

4. Compounds of the general formula I according to claim 1 wherein X = O.

5. Compounds of the general formula I according to claim 1 wherein X = CH₂.

6. Compounds of the general formula I according to claim 1:
4-(2,3'-dimethoxy-5-biphenylyl)-pyrrolidin-2-one,
4-(3',5'-dichloro-6-methoxy-3-biphenylyl)-pyrrolidin-2-one,
4-(2-methoxy-5-biphenyl-yl)-pyrrolidin-2-one,
4-(2-methoxy-4'-methyl-5-biphenylyl)-pyrrolidin-2-one,
4-(2,4'-dimethoxy-5-biphenylyl)-pyrrolidin-2-one,
4-[4-methoxy-3-(2-furyl)-phenyl]-pyrrolidin-2-one,
4-[4-methoxy-3-(2-thienyl)-phenyl]-pyrrolidin-2-one,
4-[4-methoxy-3-(3-thienyl)-phenyl]-pyrrolidin-2-one,
4-(2',4'-dichloro-6-methoxy-3-biphenylyl)-pyrrolidin-2-one,
4-(2,2'-dimethoxy-5-biphenylyl)-pyrrolidin-2-one,
4-(4'-chloro-6-methoxy-3-biphenylyl)-pyrrolidin-2-one,
4-(3'-chloro-2-methoxy-5-biphenylyl)-pyrrolidin-2-one,
4-(2-methoxy-3'-methyl-5-biphenylyl)-pyrrolidin-2-one,
4-(3'-cyclopentyloxy-2-methoxy-5-biphenylyl)-pyrrolidin-2-one,
4-(3-cyclopentyl-4-methoxyphenyl)-pyrrolidin-2-one,
4-(3-cyclohexyl-4-methoxyphenyl)-pyrrolidin-2-one,
4-(3-cycloheptyl-4-methoxyphenyl)-pyrrolidin-2-one.

7. Process for the preparation of the compounds of the general formula I, wherein the compounds of the general formula II 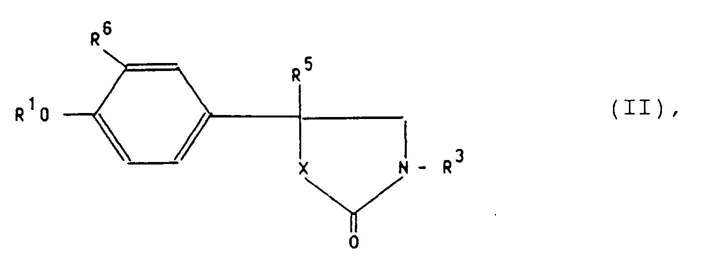 wherein R¹, R³, R⁵ and X have the above meanings and R⁶ is a leaving group,
a) are reacted in the presence of a palladium catalyst with a 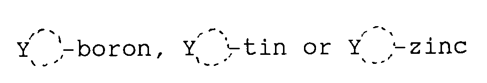 compound or with cycloalkenes, Y having the meaning given above, or
b) are converted into a lithium organyl and the latter is reacted with the appropriate ketone to form 1-hydroxycycloalkyl and, optionally, the cycloalkene is obtained by removal of water,
and then, if desired, double bonds are hydrogenated and/or NH groups are alkylated, arylated or acylated and/or ether protecting groups are removed and then, optionally, etherified and/or the isomers are separated.

8. Medicaments based on the compounds according to claims 1 to 6.

## Revendications

1. Composés de formule générale I : 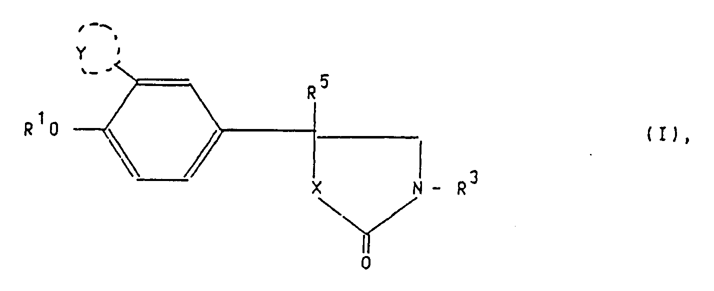 dans laquelle
R¹ désigne un alkyle en C₁₋₄,
R³ l'hydrogène, un alkyle en C₁₋₄, un acyle, un phényle,
R⁵ l'hydrogène, un alkyle en C₁₋₄,
X l'oxygène, un CH₂ ou NR⁴, R⁴ étant l'hydrogène, un alkyle en C₁₋₄,
et
Y un système cyclique aromatique ou non-aromatique, monocyclique ou bicyclique, avec de 3 à 12 atomes dans les cycles, pouvant comporter un ou deux atomes de soufre, d'oxygène et/ou d'azote et pouvant être substitué par des halogène, alkyle en C₁₋₄, NH₂ ou OR², R² désignant l'hydrogène, un alkyle en C₁₋₄, un cycloalkyle en C₃₋₇ ou un radical d'éther cyclique, ainsi que les stéréo-isomères et les mélanges.

2. Composés de formule générale I selon la revendication 1 dans lesquels R¹ est le groupe CH₃.

3. Composés de formule générale I selon la revendication 1 dans lesquels R³ est l'hydrogène.

4. Composés de formule générale I selon la revendication 1 dans lesquels X est l'oxygène.

5. Composés de formule générale I selon la revendication 1 dans lesquels X est le groupe CH₂.

6. Composés de formule générale I selon la revendication 1, à savoir :
4-(2,3'-Diméthoxy-5-biphénylyl)-pyrrolidin-2-one
4-(3',5'-Dichloro-6-méthoxy-3-biphénylyl)-pyrrolidin-2-one
4-(2-Méthoxy-5-biphénylyl)-pyrrolidin-2-one
4-(2-Méthoxy-4'-méthyl-5-biphénylyl)-pyrrolidin-2-one
4-(2,4'-Diméthoxy-5-biphénylyl)-pyrrolidin-2-one
4-[4-Méthoxy-3-(2-furyl)-phényl]-pyrrolidin-2-one.
4-[4-Méthoxy-3-(2-thiényl)-phényl]-pyrrolidin-2-one
4-[4-Méthoxy-3-(3-thiényl)-phényl]-pyrrolidin-2-one
4-(2',4'-Dichloro-6-méthoxy-3-biphénylyl)-pyrrolidin-2-one
4-(2,2'-Diméthoxy-5-biphénylyl)-pyrrolidin-2-one
4-(4'-Chloro-6-méthoxy-3-biphénylyl)-pyrrolidin-2-one
4-(3'-Chloro-2-méthoxy-5-biphénylyl)-pyrrolidin-2-one
4-(2-Méthoxy-3'-méthyl-5-biphénylyl)-pyrrolidin-2-one
4-(3'-Cyclopentyloxy-2-méthoxy-5-biphénylyl)-pyrrolidin-2-one
4-(3-Cyclopentyl-4-méthoxyphényl)-pyrrolidin-2-one
4-(3-Cyclohexyl-4-méthoxyphényl)-pyrrolidin-2-one
4-(3-Cycloheptyl-4-méthoxyphényl)-pyrrolidin-2-one

7. Procédé de préparation des composés de formule générale I caractérisé en ce que l'on fait réagir des composés de formule générale II 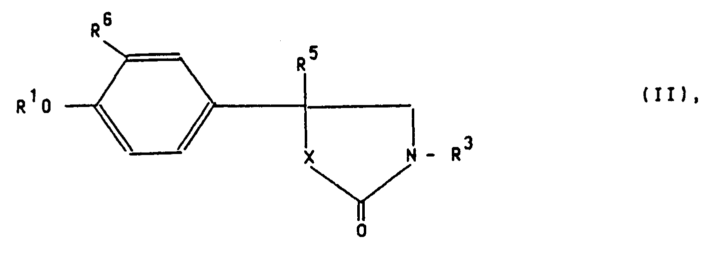 dans laquelle R¹, R³, R⁵ et X ont la signification ci-dessus et R⁶ désigne un groupe habile,
a)
en présence d'un catalyseur de palladium, avec des composés de 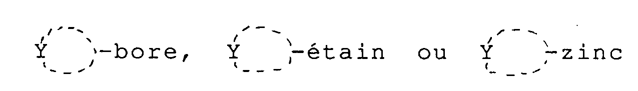 ou avec des cycloalcènes, Y ayant la signification mentionnée ci-dessus ou bien
b)
on les transforme en un organyl-lithium que l'on fait réagir avec la cétone correspondante pour former le groupe 1-hydroxy-cycloalkyle et le cas échéant on forme le cycloalcène par élimination d'eau,
puis, si l'on veut, on hydrogène les doubles liaisons et/ou on alkyle, on aryle ou on acyle les groupes NH et/ou on élimine des groupes protecteurs d'éthers, et le cas échéant on éthérifie ensuite et/ou on sépare les isomères.

8. Médicaments à base des composés selon les revendications 1 à 6.
